# EUROPÄISCHE PATENTSCHRIFT

(11) **EP 1 210 055 B1**
(45) Veröffentlichungstag und Bekanntmachung des Hinweises auf die Patenterteilung: **22.11.2006**
(21) Anmeldenummer: 00960582.5
(22) Anmeldetag: 01.09.2000
(51) Int. Cl.: A61K 6/10, C08G 73/02

(54) **ZWEIKOMPONENTIGE ELASTOMERMASSEN AUF ALKYLAZIRIDINBASIS MIT BORSÄUREKOMPLEX ENTHALTENDER KATALYSATORKOMPONENTE**
TWO-CONSTITUENT ELASTOMER MATERIALS BASED ON ALKYL AZIRIDINE COMPRISING A CATALYST CONSTITUENT THAT CONTAINS A BORIC ACID COMPLEX
MATIERES ELASTOMERES A DEUX COMPOSANTS A BASE D'ALKYLAZIRIDINE AVEC UN CONSTITUANT CATALYSEUR CONTENANT UN COMPLEXE D'ACIDE BORIQUE

(30) Priorität: 06.09.1999 DE 19942459
(43) Veröffentlichungstag der Anmeldung: 05.06.2002
(73) Patentinhaber: 3M ESPE AG, 82229 Seefeld (DE)
(72) Erfinder: ZECH, Joachim, 86916 Kaufering (DE); ECKHARDT, Gunther, 06231 Bad Düerrenberg (DE); FÜHRER, Cornelia, 87497 Wertach (DE); GANGNUS, Bernd, 82346 Andechs (DE); ROMBACH, Andreas, 82211 Herrsching (DE); GASSER, Oswald, 82229 Seefeld (DE); KLETTKE, Thomas, 86938 Schondorf (DE); WANEK, Erich, 86916 Kaufering (DE)
(86) Internationale Anmeldenummer: PCT/EP2000/008568
(87) Internationale Veröffentlichungsnummer: WO 2001/017483

(56) Entgegenhaltungen:
- EP-A- 0 279 238
- EP-A- 0 421 371
- DE-A- 19 753 456

## Beschreibung

Die Erfindung betrifft verbesserte Elastomermassen auf der Grundlage von N-Alkylaziridinoverbindungen.

Insbesondere betrifft die Erfindung solche Elastomermassen, die sich durch eine erhöhte Dehnbarkeit auszeichnen und vorzugsweise als dentale Abformmassen, Bißregistriermassen und Dubliermassen Anwendung finden.

Die Herstellung von Elastomermassen auf der Grundlage von N-Alkylaziridinopolyethern und ihre Verwendung in Dentalmaterialien ist seit langem bekannt. So beschreibt beispielsweise die DE-C-1 745 810 die Herstellung von Formkörpern auf der Basis von Aziridino-Polyethem.

In den Schriften DE-C-3 246 654, EP-A-0 421 371 und EP-A-0 110 429 ist die Verwendung von Aziridino-Polyethern in Polyetherabformmassen beschrieben.

Es ist weiterhin bekannt, daß N-Alkylaziridinoverbindungen unter Einwirkung von sauer wirkenden Verbindungen aushärten können (H. Bestian, Methoden der Organischen Chemie (Houben-Weyl), XII/1 (1958)). Die Verwendung von neutralen Schwefelsäure- oder Sulfonsäureestern als Startersubstanzen für die Aushärtung von N-Alkylaziridinoverbindungen ist in der DE-C-888 170 beschrieben.

In der DE-C-914 325 wird die Verwendung von Oxonium-, Ammonium- und Sulfoniumsalzen als Startersubstanzen vorgeschlagen.

Eine zusammenfassende Darstellung der für die Aushärtung von Aziridinoverbindungen verwendeten Startersubstanzen ist in O. C. DERMER, G. E. HAM, "Ethylenimine and other Aziridines" Academic Press (1969) enthalten.

Als prinzipiell geeignete Polymerisationsauslöser haben sich demnach eine große Anzahl von Verbindungsklassen und Verbindungen erwiesen. In der praktischen Anwendung der Aushärtung von Aziridinopolyethem ist es aber sehr schwierig, den gewünschten Abbindungsverlauf mit ausreichend langer Verarbeitungszeit und schneller Endaushärtung einzustellen. Dieses Ziel kann durch die Verwendung von speziellen Trisalkylsulfoniumsalze gemäß EP-A-0 279 238 erreicht werden.

Nachteilig bei der Verwendung von Sulfoniumsalzen als Startersubstanzen sind die bei der Aushärtung entstehenden Verbindungen, die einen unangenehmen Geruch besitzen.

Bei der Verwendung von starken Säuren als Startersubstanzen kann bei unsachgemäßer Anwendung eine Reizwirkung auf der Haut entstehen oder es können Korrosionserscheinungen an Metallen auftreten.

DE-A-19753456 offenbart zweikomponentige Elastomermassen für dentale Abformmassen enthaltend eine Basiskomponente, die N-Alkylaziridin polyether enthält und einer katalysator komponente, die Tetrafluoroborsaüre enthält.

Für die Anwendung sind die mechanischen Eigenschaften, wie Reißfestigkeit und Reißdehnung der ausgehärteten Dentalmassen von großer Bedeutung. Diese Eigenschaften liegen unter Verwendung der bekannten Startersubstanzen im allgemeinen auf einem niedrigen Niveau.

Aufgabe der Erfindung ist es, aushärtbare N-Alkylaziridinoverbindungen enthaltende Zubereitungen zur Verfügung zu stellen, bei deren Einsatz keine unangenehm riechenden Verbindungen entstehen, wobei die Anwendung starker, korrosiv wirkender Säuren vermieden wird und die ausgehärteten Dentalmassen verbesserte mechanische Eigenschaften aufweisen.

Gelöst wird diese Aufgabe durch eine verbesserte Elastomermasse auf der Grundlage von N-Alkylaziridinoverbindungen mit einer Basiskomponente, welche die Aziridinoverbindungen enthält, und mit einer Katalysatorkomponente, welche mindestens eine sauer wirkende Verbindung enthält, wobei beide Komponenten vor der Verwendung gemischt werden, dadurch gekennzeichnet, daß als eine sauer wirkende Verbindung der Katalysatorkomponente ein oder mehrere Borsäurekomplexe eingesetzt werden, die durch Umsetzung von Borsäure und/oder einem Borsäurederivat mit mindestens einer OH-funktionellen Verbindung erhältlich sind, wobei die OH-Funktionen vollständig oder teilweise geschützt vorliegen können, und wobei diese Umsetzung entweder als vorgelagerte Reaktion zwischen Borsäure und/oder einem Borsäurederivat und mindestens einer solchen OH-funktionellen Verbindung oder während oder nach der Herstellung der Katalysatorkomponente oder durch Mischung der Katalysatorkomponente mit der Basiskomponente, die dann mindestens eine solche OH-funktionelle Verbindung enthält, erfolgt, und ausgewählt ist aus
(i) organischen Verbindungen, die mindestens eine 1,2-Dihydroxy- und/oder 1,3-Dihydroxygruppe enthalten,
(ii) Verbindungen, die mindestens eine phenolische OH-Gruppe besitzen,
(iii) α-Hydroxycarbonsäuren,
wobei die OH-funktionelle Verbindung die allgemeine Strukturformel 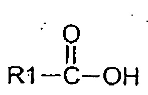 und bzw. oder 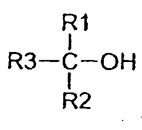 aufweist, wobei R1, R2 und R3 die gleichen oder verschiedene Reste repräsentieren,
die Wasserstoff, aliphatische, cycloaliphatische, aromatische oder araliphatische Substituenten mit 1 bis 30 C-Atomen darstellen, in denen jeweils ein oder mehrere C-Atome durch -O-; -S-; 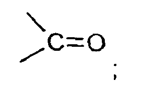 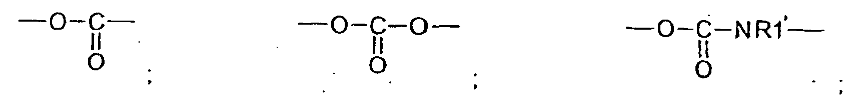 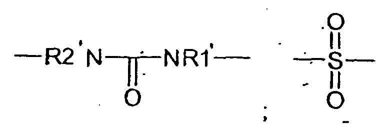 ersetzt sein können, wobei R1' und R2' gleich oder verschieden und aliphatische; cycloaliphatische, aromatische oder araliphatische zweiwertige Reste mit 1 bis 30 C-Atomen sind.

Vorzugsweise besteht die Elastomermasse nur aus der Basiskomponente und der Katalysatorkomponente.

Die erfindungsgemäßen verbesserten Elastomermassen werden beispielsweise als dentale Abformmassen, als Bißregistriermassen und als Dubliermassen eingesetzt.

Die ausgehärteten Massen weisen überraschenderweise auch stark verbesserte mechanische Eigenschaften auf. Offensichtlich können durch die Wahl der Startersubstanzen die mechanischen Eigenschaften, wie Reißfestigkeit und Reißdehnung der ausgehärteten Massen, beeinflußt werden.

Die als Bestandteil der Katalysatorkomponente verwendete Borsäure bzw. die verwendeten Borsäurederivate werden zu einem Massenanteil von 0,1 bis 100 % der Katalysatorkomponente eingesetzt.

Günstige Ergebnisse hinsichtlich der Verbesserung der mechanischen Eigenschaften sind erreichbar, wenn das Verhältnis von Molanzahl Borsäure zur Zahl der Aziridinoäquivalente in der aushärtenden Masse 1 : 1 bis 1 : 20, bevorzugt 1 : 1,2 bis 1 : 10 und besonders bevorzugt 1 : 1,5 bis 1 : 6 beträgt, wobei die Aziridinoäquivalentmasse der verwendeten N-Alkylaziridinoverbindungen im Bereich von 500 bis 25000 g / Äquivalent, bevorzugt im Bereich von 1000 bis 8000 g / Äquivalent und besonders bevorzugt im Bereich von 2000 bis 6000 g / Äquivalent liegt.

Es ist seit langem bekannt, daß Polyol-Verbindungen, wie Zucker, Zuckeralkohole, Zuckersäuren und Uronsäuren esterartige Komplexe mit Borsäure bilden. Eine Zusammenfassung der analytisch relevanten Ergebnisse ist in "Treatise on Analytical Chemistry", Part II, Vol. 10, Wiley, New York enthalten.

Diese Komplexbildung ist mit einer Aciditätserhöhung verbunden und gestattet die einfach zu realisierende alkalimetrische Titration von Borsäure.

Die Komplexbildung wird aber auch in chromatographischen und conductimetrischen Analysenverfahren und in Prozessen der technischen Extraktion von Borsäure aus diese enthaltenden wäßrigen Gemischen genutzt.

Gut untersuchte Komplexbildner für Borsäure im Hinblick auf die alkalimetrische Titration sind Fructose, Glycose, Mannit, Sorbit und Glycerin; die lonisationskonstanten und die Beständigkeitskonstanten der jeweiligen Komplexe sind angegeben (W. A. Nasarenko et al., Zaw. Lab. 34 (1968), 257).

Überraschenderweise wurde gefunden, daß Borsäurekomplexe mit OH-funktionalisierten Verbindungen in der Lage sind, die Aushärtung von N-Alkylaziridinoverbindungen bei Raumtemperatur und in nutzbarer Geschwindigkeit zu bewirken, was mit Brönsted-Säuren, die den etwa gleichen pKₛ-Wert wie diese Komplexe besitzen, meist nicht der Fall ist.

Völlig überraschend war auch der Befund, daß durch den Einsatz von ausgewählten Polyol-Borsäure-Komplexen das Niveau der mechanischen Eigenschaften deutlich verbessert werden kann.

Weiterhin wurde gefunden, daß sowohl bei der Bildung der Komplexe als auch während der Aushärtung der N-Alkylaziridinopolyether keine unangenehm riechenden Verbindungen entstehen.

Durch die Wahl der Komplexbildner kann ferner die Reizwirkung der Katalysatorkomponente auf der Haut, beispielsweise bei nicht sachgerechtem Umgang, als auch die korrosive Wirkung auf unedle Metalle stark verringert bzw. vermieden werden.

Die im Zuge der Erfindung einzusetzenden Umsetzungsprodukte von Borsäure mit OH-haltigen Verbindungen, vereinfacht "Polyol-Borsäure-Komplexe" genannt, werden durch Umsetzung von Borsäure oder Borsäurederivaten mit Verbindungen hergestellt, die bevorzugt mindestens zwei OH-Gruppen enthalten, wobei bei der Komplexierung auch Verbindungen mit nur einer OH-Gruppe verwendet werden können.

Erfindungsgemäß werden dabei OH-funktionelle Verbindungen eingesetzt, die mindestens eine und bis zu 10 OH-Gruppen der allgemeinen Strukturformel 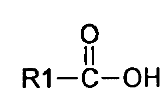 und bzw. oder 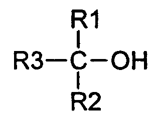 enthalten, wobei R1, R2 und R3 die gleichen oder verschiedene Reste repräsentieren, die Wasserstoff, aliphatische, cycloaliphatische, aromatische oder araliphatische Substituenten mit 1 bis 30 C-Atomen darstellen, in denen jeweils ein oder mehrere C-Atome durch -O- ; -S-; 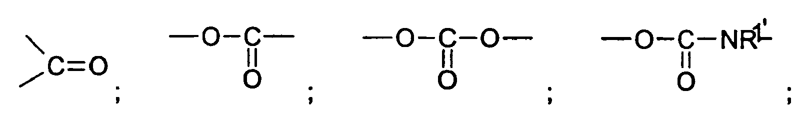 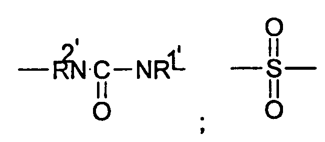 ersetzt sein können, wobei R^{1'} und R^{2'} gleich oder verschieden und aliphatische, cycloaliphatische, aromatische oder araliphatische zweiwertige Reste mit 1 bis 30 C-Atomen sind.

In den verwendeten Komplexbildnern mit zwei OH-Gruppen liegen diese in 1,2-Stellung, in 1,3-Stellung vor.

Bei Verwendung von Alkoholen mit mehr als zwei OH-Gruppen sind unterschiedliche Stellungen dieser OH-Gruppen möglich. Günstig ist die 1, 2, 3-Stellung, wie sie in einigen Zuckern vorliegt.

Mit Vorteil werden Komplexbildner eingesetzt, die mindestens eine phenolische OH-Gruppe besitzen. Hierbei sind solche Verbindungen besonders bevorzugt, die zusätzlich mindestens noch eine weitere phenolische OH-Gruppe oder eine aliphatische OH-Gruppe und ggf. weitere Substituenten am aromatischen Kern tragen.

Eine weitere Gruppe der Komplexbildner enthält eine aliphatische OH-Gruppe in alpha-Stellung zur Carboxylgruppe tragen.

Typische Vertreter der Komplexbildner für den erfindungsgemäßen Einsatz sind:
1. Glycerin und seine Ether- oder Ester-Derivate, sowie alkoxy-verlängerte Glycerine und Polyglycerine, wie beispielsweise Diglycerin, Tetraglycerin, Glycerinpropoxylat,
2. Alkylenglykole, wie Ethylenglykol und Propylenglykol,
3. Mono- und Multi-1,2-diole mit Alkylresten oder Alkylenbrücken, wie beispielsweise 1,2-Hexandiol, 1,2-Cyclohexandiol, 3-Chlorpropan-1,2-diol, 1,2-Propandiol, 3-Mercapto-1,2-Propandiol, Pinakol, 3-Brompropan-1,2-diol, 1,2-Butandiol und 1,2,9,10-Tetrahydroxydecan,
4. Mono- und Multi-1,3-diole mit Alkylresten oder Alkylenbrücken, wie 1,3-Butandiol, 2-Ethyl-1,3- hexandiol, 2-Ethyl-2-butylpropan-1,3-diol, 1,3-Butandiol, 1,3-Cyclohexandiol, 1,3-Hexandiol, 2,2-Diethylpropan-1,3-diol, 2,4-Pentandiol, 2-Methyl-2-propyl-1,3-propandiol, 2,2-Dimethylpropan-1,3-diol, sowie Diole, wie 1,5-Butandiol, 1,6-Hexandiol, cis-2-Buten-1,4-diol, 2-Butin-1,4-diol, und alpha,omega-OH-funktionalisierte Polymere, wie Poly-THF,
5. Polyalkohole, wie Glucose, Fructose, Lactose, Arabinose, Ribose, Xylose, Mannose, Galactose, Sorbose, Xylulose, Ribulose, Mannit, Sorbit, Maltitol, Lactitol, Gluconitril, Pentaerythrit, Threit, Erythrit, Arabit,
6. Hydroxycarbonsäuren, wie Gluconsäure, 2-Ketogluconsäure, Mannozuckersäure, Schleimsäure, Glucuronsäure, Chinasäure, Weinsäure, Ascorbinsäure, Mandelsäure, 4-Chlormandelsäure, Milchsäure, Glycolsäure, Benzilsäure, Vinylglycolsäure, Zitronensäure, Phenylmilchsäure, Tropasäure, Atrolactinsäure, Dihydroxyfumarsäure, Glycolsäure, Hydroxymalonsäure, 2-Hydroxybernsteinsäure, Tartronsäure, Salicylsäure,
7. Ester von Hydroxycarbonsäuren, wie Gluconsäureethylester, Weinsäurediethylester, Weinsäuredimethylester, Weinsäuredibutylester, Glycolsäureethylester, Xylitdimethacrylat,
8. Phenolische Verbindungen, wie Brenzcatechin, 4-tert.-Butylbrenzcatechin, 3,5-Di-tert.-butylbrenzcatechin, Pyrogallol, Salicylalkohol, 3-Methoxybrenzcatechin, 2,3-Dihydroxynaphthalin, 5-Bromsalicylalkohol, 5-Chlorsalicylalkohol, 4-Chlorsalicylalkohol, 3-Chlorsalicylalkohol, 3,5-Dichlorsalicylalkohol.

Die Verwendung von mehreren solchen OH-funktionellen Verbindungen ist möglich und kann zweckmäßig sein, um spezielle Eigenschaften, wie beispielsweise einen gewünschten zeitlichen Aushärtungsverlauf einzustellen.

Das molare Verhältnis zwischen Borsäure und den OH-funktionellen Verbindungen kann in einem weiten Bereich von 1 : 0,1 bis 1 : 10 variiert werden, wobei der Bereich von 1 : 1 bis 1 : 4 bevorzugt ist.

Das für den jeweiligen Anwendungsfall optimale Verhältnis ist abhängig von der jeweilig notwendigen Konzentration und der Löslichkeit des Komplexes, der Gleichgewichtslage und der Auswirkung eines Überschusses an Komplexierungsmittel auf die Eigenschaften der Dentalmassen.

Anstelle von Borsäure können auch ganz oder teilweise Borsäurederivate, wie beispielsweise Borsäureanhydrid, Borate und Borsäure-C₁₋₁₈-, vorzugsweise -C₂₋₄-ester, eingesetzt werden.

Weiterhin ist die Anwendung der erfindungsgemäßen Starter für die Aushärtung der N-Alkylaziridinoverbindungen auch in Kombination mit bekannten anderen Startersubstanzen, wie Brönstedsäuren oder Sulfoniumsalzen möglich, wobei die beschriebenen negativen Wirkungen der letztgenannten Starter verringert und ihre positiven Wirkungen, wie beispielsweise gute Einstellbarkeit des Aushärtungsverlaufs genutzt werden.

Für die Realisierung der erfindungsgemäßen Verwendung der Borsäurekomplexe als Startersubstanzen der Polyether-Aushärtung gibt es verschiedene Varianten.

Nach der ersten Variante wird die Umsetzung vor der Formulierung der Katalysatorkomponente durchgeführt und der esterartige Borsäurekomplex als Bestandteil der Katalysatorkomponente eingesetzt.

Die Herstellung des Komplexes erfolgt in an sich bekannter Weise, beispielsweise unter Zusatz von Toluol als Schleppmittel für das durch Veresterung entstehende Wasser.

Nach einer zweiten Variante wird die Komplexierung der Borsäure durch Mischung von Borsäure oder eines Borsäurederivats mit der oder den OH-funktionalisierten Verbindungen während der Formulierung der Katalysatorkomponente bewirkt, wobei das durch Veresterung entstehende Wasser entweder in der Katalysatorkomponente verbleibt oder durch geeignete Maßnahmen, wie beispielsweise eine Vakuumbehandlung der Katalysatorkomponente, ganz oder teilweise entfernt wird.

Üblicherweise findet die Formulierung der Katalysatorkomponente bei Temperaturen im Bereich von 20 bis 50°C statt, die Anwendung höherer Temperaturen kann zur Beschleunigung der Komplexierung bzw. zur Einstellung des gewünschten Wassergehaltes zweckmäßig sein.

Bei Anwendung der ersten und zweiten Varianten kann die Basiskomponente ggf. zusätzlich ein neutrales oder basisch wirkendes Borsäurederivat, wie beispielsweise Alkalisalze der Borsäure oder Trialkylester der Borsäure enthalten.

Nach einer dritten Variante wird der die Aushärtung startende Komplex erst nach Mischung der Katalysatorkomponente mit der Basiskomponente gebildet, wobei bevorzugt die Katalysatorkomponente die Borsäure oder ein Borsäurederivat und die Basiskomponente den Komplexbildner enthält.

Nach einer besonderen Ausführungsform der dritten Variante liegt die Borsäure im Katalysator in einer komplexierten oder veresterten Form vor; nach der Mischung mit der Basiskomponente erfolgt eine Umkomplexierung.

Nach einer weiteren Ausführungsform der dritten Variante wird die Komplexierung der Borsäure durch eine oder mehrere Verbindungen bewirkt, bei der die OH-Gruppen dieser Verbindungen derivatisiert sind. Beim Vermischen der beiden Komponenten werden die reaktiven Borsäurekomplexe gebildet.

Eine Derivatisierung der OH-Gruppen kann beispielsweise durch Veresterung, Veretherung oder Silylierung erreicht werden, wobei die Silylierung bevorzugt ist.

Die silylierten Komplexbildner können sowohl in der Basiskomponente als auch in der Katalysatorkomponente eingesetzt werden.

Die aufgeführten Varianten und deren Ausführungsformen können auch in Kombination untereinander angewendet werden. Solche Kombinationen können sich als günstig erweisen, um die Lagerstabilität der Katalysator- und Basiskomponenten zu erhöhen.

Zur Erreichung von bestimmten mechanischen Eigenschaften oder zur Erzielung eines gewünschten Abbindeverlaufes kann es zweckmäßig sein, Kombinationen von Borsäurekomplexen mit unterschiedlicher Struktur und Zusammensetzung einzusetzen.

Üblicherweise enthalten die Katalysatorkomponenten nach der bevorzugten ersten und zweiten Variante:
(A) 0,1 bis 100 Gew.-% mindestens eines Borsäurekomplexes, ggf. in einem Überschuß an Komplexierungsmittel,
(B) 0 bis 95 Gew.-% mindestens eines inerten Verdünnungsmittels,
(C) 0 bis 80 Gew.-% von Modifikatoren, einschließlich Füllstoffen, Farbstoffen, Pigmenten, Thixotropiemitteln, Fließverbesserem, polymeren Eindickem, oberflächenaktiven Substanzen, Stabilisatoren, polymerisationsverzögernden Verbindungen, Geruchsstoffen und Geschmacksstoffen,
   wobei die Gew.-%-Angaben jeweils auf die Gesamtmasse der Katalysatorkomponente bezogen sind;
   und die Basiskomponenten:
(D) 5 bis 100 Gew.-% eines Gemisches von N-Alkylaziridinoverbindungen mit Aziridinoäquivalentmassen von 500 bis 25000 g / Äquivalent, bevorzugt im Bereich von 1000 bis 8000 g / Äquivalent und besonders bevorzugt im Bereich von 2000 bis 6000 g / Äquivalent ,
(E) 0 bis 95 Gew.-% mindestens eines inerten Verdünnungsmittels,
(F) 0 bis 80 Gew.-% von Modifikatoren, einschließlich Füllstoffen, Farbstoffen, Pigmenten, Thixotropiemitteln, Fließverbesserem, polymeren Eindickem, oberflächenaktiven Substanzen, Stabilisatoren, polymerisationsverzögernden Verbindungen, Geruchsstoffen und Geschmacksstoffen,
wobei die Gew.-%-Angaben jeweils auf das Gesamtgewicht der Basiskomponente bezogen sind;
und wobei die Komponenten getrennt gelagert und zur Verarbeitung in einem Verhältnis von Katalysatorkomponente zu Basiskomponente von 5 : 1 bis 1 : 20, vorzugsweise von 1 : 1 bis 1 : 10, miteinander gemischt werden.

Als inerte Verdünnungsmittel entsprechend den Bestandteilen (B) und (E) können Polyetherpolyole, wie beispielsweise Polypropylenglykole oder Mischpolyetherole mit Tetrahydrofuran- und bzw. oder Ethylenoxid- und bzw. oder Propylenoxid-Einheiten, Polyesterpolyole, wie beispielsweise Polycaprolactondiole und Polycaprolactontriole, Polycarbonatdiole, aliphatische Ester, Öle, Fette, Wachse, aliphatische Kohlenwasserstoffe, araliphatische Kohlenwasserstoffe sowie ein- oder mehrfunktionelle Ester von mehrwertigen Säuren wie beispielsweise Phthalsäure, Adipinsäure oder Zitronensäure oder Ester oder Amide von Alkylsulfonsäuren und Arylsulfonsäuren verwendet werden.

Der Bestandteil (B) bzw. (E) wird in Mengen von 0 bis 95 Gew.-%, vorzugsweise 10 bis 90 Gew.-% und besonders bevorzugt 40 bis 85 Gew.-%, bezogen auf das Gesamtgewicht der Katalysatorkomponente bzw. der Basiskomponente eingesetzt.

Als Verbindungen entsprechend den Bestandteilen (B) bzw. (E), können organische Verbindungen eingesetzt werden, die die Gesamtmischung hydrophobieren und völlig unterschiedlichen Verbindungsklassen angehören.

Gute Ergebnisse werden mit Kohlenwasserstoffen mit 6 bis 30 C-Atomen erzielt, die in der Basiskomponente gelöst werden oder in stabiler feinteiliger Form einarbeitbar sind. Die Kohlenwasserstoffe können aliphatisch und bzw. oder aromatisch sowie olefinisch sein und in verzweigter und bzw. oder linearer Form vorliegen.

Typische Beispiele sind Polypropylenöle oder Polyisobutylenöle. Mit Vorteil werden aromatische Kohlenwasserstoffe, wie beispielsweise Polyphenylenverbindungen, Dibenzyltoluol und Dibenzylphenylmethan, eingesetzt.

Weiterhin sind wachsartige Verbindungen mit Esterstrukturen verwendbar. Typische Vertreter dieser Verbindungsklasse sind die Esterwachse, wie sie beispielsweise von der Fa. Hoechst unter der Bezeichnung Hoechst-Wachs E; F; X 22 vertrieben werden.

Der Katalysatorkomponente wie auch der Basiskomponente können Modifikatoren, entsprechend den Bestandteilen (C) bzw. (F), in einem weiten Konzentrationsbereich zugesetzt werden. Die Bestandteile (C) bzw. (F) werden in Mengen von 0 bis 80 Gew.-%, vorzugsweise von 0 bis 50 Gew.-% und besonders bevorzugt jeweils von 15 bis 40 Gew.-%, bezogen auf das Gesamtgewicht der Katalysatorkomponente bzw. Basiskomponente, eingesetzt.

Diese Modifikatoren sind meist feinteilige Füllstoffe wie Alumosilikate, Kieselsäuren, Quarzmehl, Wollastonit, Glimmermehl und Diatomeenerde sowie Farbstoffe und Pigmente, deren Zusatz eine bessere Beurteilung der Mischgüte ermöglicht und die Verwechslungsgefahr vermindert, Thixotropiemittel wie feindisperse Kieselsäuren und andere das Fließverhalten beeinflussende Zusätze, wie polymere Eindicker, weiterhin oberflächenaktive Substanzen zur Einstellung des Anfließverhaltens sowie Geruchsstoffe und Geschmacksstoffe.

Als Bestandteil (D) der Basiskomponente werden Gemische von N-Alkylaziridinoverbindungen verwendet, wobei die Aziridinoäquivalentmassen von 500 bis 25000 g / Äquivalent variiert werden können und die Anzahl der N-Alkylaziridinogruppen zwischen 1 und 4 pro Molekül variiert werden kann.

Bevorzugt werden Gemische von N-Alkylaziridinopolyethern eingesetzt, die zumindest zu 60 % aus Polyetherverbindungen bestehen, die mindestens zwei Aziridinogruppen tragen. Nach einer anderen bevorzugten Ausführungsform der Erfindung werden Gemische von N-Alkylaziridinopolyethern verwendet, die zumindest zu 5 % aus Polyetherverbindungen, die mindestens 3 Aziridinogruppen enthalten, bestehen.

Als Polyethergrundkörper sind solche mit Tetrahydrofuran- und bzw. oder Ethylenoxid- und bzw. oder Propylenoxid-Einheiten einsetzbar.

Bevorzugt besteht das Gemisch der N-Alkylaziridinopolyether aus Mischpolyetherderivaten von Ethylenoxid und Tetrahydrofuran, eingebaut in einem molaren Verhältnis von 1 : 2 bis 1 : 5, bevorzugt von 1 : 3 bis 1 : 4.

Der Bestandteil (D) wird in Konzentrationen von 5 bis 100 Gew.%, vorzugsweise 20 bis 70 Gew.-% und besonders bevorzugt 30 bis 60 Gew.-%, bezogen auf das Gesamtgewicht der Basiskomponente, eingesetzt.

Zur Einstellung des gewünschten Abbindeverlaufs können die erfindungsgemäßen Zubereitungen gemäß Bestandteil (C) bzw. (F) mindestens eine die Aushärtung verzögernde Verbindung enthalten. Prinzipiell verzögern aminische oder alkalische Substanzen die Aushärtung der N-Alkylaziridinoverbindungen und können für diesen Zweck eingesetzt werden.

So beschreibt die DE-A1-197 534 61, auf die hier vollinhaltlich Bezug genommen wird, den Einsatz von 0,0005 bis 50 Gew.-% an löslichen und/oder feinteiligen Erdalkali- und/oder Alkalimetallverbindungen.

So können beispielsweise der Katalysatorkomponente und bzw. oder der Basiskomponente Lösungen von Lithiumverbindungen, wie Lithiumhydroxid oder Lithiumcarbonat, zugesetzt werden. Der Einsatz von Lithiumcarboxylaten ist ebenfalls möglich.

Die erfindungsgemäßen zweikomponentigen Zubereitungen auf der Basis von N-Alkylaziridinoverbindungen können in Abhängigkeit von der Zusammensetzung der Katalysatorkomponente und der Basiskomponente für das Verkleben von Substraten, für das Abdichten, das Beschichten und das Vergießen eingesetzt werden.

Jedoch werden die erfindungsgemäßen Zubereitungen bevorzugt zur Abformung von Gegenständen eingesetzt, wobei mit den Zubereitungen gemäß der Erfindung aufgrund ihres hervorragenden Anfließverhaltens detailgetreue Abformungen erhalten werden.

Mit besonderem Vorteil werden die Zubereitungen gemäß der Erfindung bei der zahnmedizinischen Abformung und der zahntechnischen Dublierung eingesetzt.

Bei der zahnmedizinischen Abformung erweist sich das gute Anfließverhalten an den feuchten Zahn und das feuchte Zahnfleisch sowie die Unempfindlichkeit der Präzision der Abformung gegenüber Speichel und Blut als großer Vorteil.

Bei der zahntechnischen Dublierung ist das gute Anfließverhalten an hydrophile Gipsoberflächen und die gute Benetzbarkeit der erhaltenen Dublierungen mit Gipsbrei oder angemischten Einbettmasse-Formulierungen vorteilhaft.

Dabei kann die Dosierung der beiden Komponenten nach Sicht, beispielsweise über den sog. Stranglängenvergleich, nach Gewicht, über vordosierte Packungseinheiten und nachfolgende Handanmischung, aus Doppelkammerkartuschen mit statischem Mischrohr oder mittels Volumendosieranlagen mit nachgeschaltetem statischen oder dynamischen Mischer erfolgen.

Zur Erzielung optimaler Ergebnisse ist eine hohe Mischgüte erforderlich. Dagegen ist die Toleranz des Mischungsverhältnisses im allgemeinen relativ groß und kann beispielsweise bei einem vorgegebenen Verhältnis von Katalysatorkomponente zu Basiskomponente von 1 : 5 den Bereich 0,75 bis 1,25 : 5 umfassen, ohne daß einsatzbeschränkende Eigenschaftsänderungen feststellbar sind.

Die Erfindung wird durch die nachfolgenden Beispiele näher beschrieben, ohne daß sie dadurch beschränkt werden soll.

### Beispiele

### 1. Herstellung und Ausprüfung von Abformmassen

Mit Hilfe von Laborknetem wurden die in Tabelle 1 beschriebenen Katalysatorkomponenten im 100-g-Maßstab hergestellt. Die Herstellung der Basiskomponenten, die in Tabelle 2 beschrieben sind, erfolgte im 500-g-Maßstab.

In Tabelle 3 sind die Mischungen zusammengestellt, die unter Verwendung der in Tabelle 1 beschriebenen Katalysatorkomponenten und der in Tabelle 2 beschriebenen Basiskomponente im jeweils angegebenen Gewichtsverhältnis untersucht wurden. Die Mischungen wurden durch Anspateln auf den Mischblock innerhalb von 30 Sekunden zubereitet und zur Bestimmung der ebenfalls in Tabelle 3 zusammengestellten Eigenschaften eingesetzt.

Die Mundentnahmezeit konnte als Mittelwert von jeweils 3 Abdrucknahmen an 3 verschiedenen Probanden in Form eines vollständigen Oberkiefer-Abdruckes ermittelt werden.

Alle Mischungen der erfindungsgemäßen Beispiele 1 bis 10 (Tabelle 3) ergaben Abdrücke, die nach der Entnahme aus dem Mund nicht klebrig waren und sich durch eine sehr gute Zeichnungsschärfe auszeichneten.

Die Einzelkomponenten und auch die Mischungen zeigten keinen unangenehmen oder auffälligen Geruch.

### 2. Herstellung und Ausprüfung von Dubliermassen

Die in Tabelle 4 beschriebenen Katalysatorkomponenten wurden im 100-g-Maßstab durch Mischen der organischen Komponenten und Einarbeiten der Füllstoffe mit Hilfe eines Dissolvers und das Homogenisieren in einem Laborkneter hergestellt.

Die Herstellung der in Tabelle 5 beschriebenen Basiskomponenten erfolgte in analoger Weise im 100-g-Maßstab, wobei vor dem Einarbeiten der Füllstoffe das verwendete organische Thixotropieadditiv (Thixatrol ST) bei 55°C - 60°C eingelöst wurde.

Die beim Mischen der Komponenten im Gewichtsverhältnis 1 : 1 erhaltenen Eigenschaften der Dubliermassen sind in Tabelle 6 zusammengestellt.

Die Dubliermassen gemäß den Erfindungsbeispielen 11 bis 16 zeichneten sich durch eine hervorragende Zeichnungsschärfe, ein sehr gutes Anfließen an hydrophile Gipsoberflächen und eine ausgezeichnete Benetzung der erhaltenen Dublierungen mit Gipsbrei bzw. der angemischten Einbettmasse aus.

### 3. Herstellung und Ausprüfung von Bißregistriermaterialien

Die in Tabelle 7 beschriebenen Katalysatorkomponenten sowie die in Tabelle 8 beschriebenen Basiskomponenten wurden im 100-g- bzw. 500-g-Maßstab in Laborknetem hergestellt.

Tabelle 9 enthält die Charakterisierung der Mischungen, die durch Anspateln auf dem Mischblock innerhalb von 25 Sekunden hergestellt wurden.

Weiterhin enthält Tabelle 9 die Verarbeitungszeit der erhaltenen Mischungen bei 23° C, die Mundentnahmezeit, jeweils gemessen ab Mischungsbeginn und die Shore-A-Härte nach 24 Stunden.

Die Bißregistrierrate gemäß den Erfindungsbeispielen 17 bis 20 zeichneten sich durch eine sehr hohe Präzision aus und waren gut beschneidbar und fräsbar.

**Tabelle 1**

| Zusammensetzung der Katalysatorkomponenten für dentale Abformmassen | | | | | | |
|---|---|---|---|---|---|---|
| **Bestandteil** | **Bezeichnung der Katalysatorkomponenten** | | | | | |
| | **Gew.-%** | | | | | |
| | **AM-K1** | **AM-K2** | **AM-K3** | **AM-K4** | **AM-K5** | **AM-K6** |
| Borsäure | 3,72 | 3,57 | 3,81 | - | 1,10 | 3,70 |
| Salicylalkohol | 16.00 | 15,70 | 21,00 | - | 5,90 | - |
| Mandelsäure | - | - | - | - | - | 19,60 |
| Umsetzungsprodukt von Borsäure mit Salicylalkohol im Molverhältnis 1 : 2 | - | - | - | 15,30 | 12,70 | - |
| Fällungskieselsäure (Sipernat D17) | 20,30 | 21,50 | 20,00 | 21,00 | 23,70 | 20,20 |
| Statistisches Mischpol-etherdiol, hergestellt aus Ethylenoxid und Propylenoxid mit einer Molmasse von 3200 g / Mol | - | 58,53 | - | - | 25,30 | - |
| Polypropylenoxid-diol mit einer Molmasse von 2000 g / Mol | - | - | 54,41 | 63,00 | 30,60 | 55,80 |
| Polypropylenoxid-diol mit einer Molmasse von 4000 g / Mol | 59,28 | - | - | - | - | - |
| Lithiumhydroxid | | | 0,08 | | | - |
| Farbpaste, rot | 0,70 | 0,70 | 0,70 | 0,70 | 0,70 | 0,70 |

**Tabelle 2**

| Zusammensetzung der Basiskomponenten für dentale Abformmassen | | | |
|---|---|---|---|
| **Bestandteil** | **Gew.-%** | | |
| | **AM-B1** | **AM-B2** | **AM-B3** |
| Gemisch aus Bisaziridinopolyethern mit einer mittleren Iminoäquivalentmasse von 3100, hergestellt aus einem Polyetherdiol, das aus Ethylenoxid- und Tetrahydrofuran-Einheiten im Molverhältnis 1 : 3,5 besteht, mit einem Gehalt an cyclischen Polyethern von 0,27 % | 55,70 | 58,11 | 54,97 |
| Diatomeenerde (Celatom MW 25) | 10,69 | 11,50 | 14,00 |
| Hydriertes Pflanzenöl | 13,91 | 12,51 | 14,72 |
| Dibenzyltoluol | 17,80 | 9,08 | 9,41 |
| Statistisches Mischpolyetherdiol, hergestellt aus Ethylenoxid und Propylenoxid mit einer Molmasse von 3200 g / Mol | - | 6,90 | - |
| Polypropylenoxiddiol mit einer Molmasse von 2000 g / Mol | - | - | 5,00 |
| Farbpaste, grau | 1,90 | 1,90 | 1,90 |

**Tabelle 3**

| Erfindungsgemäße Elastomermassen unter Verwendung der Katalysatorkomponenten gemäß Tabelle 1 und von Basiskomponenten gemäß Tabelle 2 und ermittelte Eigenschaften | | | | | | | | | | |
|---|---|---|---|---|---|---|---|---|---|---|
| | **Erfindungsbeispiele-Nr.** | | | | | | | | | |
| | **1** | **2** | **3** | **4** | **5** | **6** | **7** | **8** | **9** | **10** |
| Katalysatorkomponente | AM-K1 | AM-K1 | AM-K2 | AM-K2 | AM-K2 | AM-K3 | AM-K4 | AM-K5 | AM-K5 | AM-K6 |
| Basiskomponente | AM-B1 | AM-B2 | AM-B1 | AM-B2 | AM-B3 | AM-B1 | AM-B3 | AM-B1 | AM-B2 | AM-B1 |
| Mischungsverhältnis (nach Gewicht) K : B | 1 : 4,7 | 1:5,4 | 1 :4,9 | 1 :5,1 | 1 : 4,6 | 1:5,0 | 1:5,1 | 1 : 5,2 | 1 :4,8 | 1 : 5,0 |
| Aushärtungsbeginn bei 23° C (Sekunden) | 120 | 120 | 130 | 125 | 135 | 150 | 125 | 145 | 140 | 40 |
| Mundentnahmezeit (Sekunden) | 260 | 245 | 280 | 270 | 290 | 275 | 280 | 250 | 240 | 120 |
| Reißdehnung / % | 235 | 255 | 246 | 257 | 242 | 285 | 320 | 380 | 360 | 140 |
| Reißfestigkeit / MPa | 1,65 | 1,75 | 1,89 | 1,85 | 1,95 | 1,84 | 1,90 | 2,01 | 2,10 | 1,35 |
| Shore A-Härte nach 24 h | 48 | 52 | 49 | 51 | 53 | 49 | 50 | 48 | 51 | 50 |

**Tabelle 4**

| Zusammensetzung und Viskosität der Katalysatorkomponenten für Dubliermassen | | | | | | |
|---|---|---|---|---|---|---|
| **Bestandteil** | **Gew.-%** | | | | | |
| | **DM-K1** | **DM-K2** | **DM-K3** | **DM-K4** | **DM-K5** | **DM-K6** |
| Borsäure | 0,65 | 0,63 | 0,66 | - | 0,65 | 0,72 |
| 4-tert.-Butylbrenzcatechin | 3,49 | 3,42 | 3,55 | - | - | - |
| Brenzcatechin, silanisiert mit Dichlordimethylsilan | - | - | - | - | 7,00 | - |
| Umsetzungsprodukt von Borsäure mit Brenzcatechin im Molverhältnis 1 : 1,95 | - | - | - | 2,40 | - | - |
| 1,6-Hexandiol | 4,00 | 4,50 | - | - | 4,50 | - |
| L-(+)-Weinsäurediethylester | - | 6,00 | - | - | - | |
| cis-1, 4-Butendiol | - | - | 5,86 | - | - | - |
| Glykolsäure | - | - | - | - | - | 0,8 |
| Glycerin | - | - | - | - | - | 1,65 |
| Alkylsulfonsäureester des Phenols (Mesamoll) | 11,86 | 5,45 | 29,22 | 13,60 | 9,85 | 18,83 |
| Phthalsäurepolyester (Ultramoll PP) | 66,00 | 66,00 | 47,00 | 70,00 | 66,00 | 66,00 |
| Fällungskieselsäure (Sipernat D10) | - | - | 4,90 | - | - | - |
| Fällungskieselsäure (Sipernat 22S) | 10,00 | 10,00 | 8,80 | - | - | 6,00 |
| Pyrogene Kieselsäure (HDK H 2000) | 4,00 | 4,00 | - | 14,00 | 12,00 | 6,00 |
| Farbpigment, gelb (Thermoplastgelb 084F) | - | - | 0,01 | - | - | - |
| Viskosität der Katalysatokomponente / mPas | 3020 | 2480 | 1980 | 2250 | 2530 | 2620 |

**Tabelle 5**

| Zusammensetzung und Viskositäten der Basiskomponenten für Dubliermassen | | | |
|---|---|---|---|
| **Bestandteil** | **Gew.-%** | | |
| | **DM-B1** | **DM-B2** | **DM-B3** |
| Gemisch aus Bisaziridinopolyethern mit einer mittleren Iminoäquivalentmasse von 3120, hergestellt aus einem Polyetherdiol, das aus Ethylenoxid- und Tetrahydrofuran-Einheiten im Molverhältnis 1 : 3,4 besteht | 40,00 | 39,70 | 45,00 |
| Diatomeenerde (Celatom MW 25) | 16,00 | 15,30 | 10,00 |
| Dioctyladipat (Plastomoll DOA) | 38,24 | 42,4998 | 40,50 |
| Blockmischpolyether (Synperonic PE / L 121) | 5,00 | - | 2,50 |
| Modifiziertes Rizinusölderivat (Thixatrol ST) | 0,75 | 1,00 | - |
| Modifiziertes Schichtsilikat (Bentone SD-3) | - | - | 2,00 |
| Farbpaste, weiß (Lithopone) | - | 1,50 | - |
| Farbstoff, violett (Makrolex Violett B) | 0,01 | - | - |
| Farbstoff, blau (Thermoplast Blau 684) | - | 0,0002 | - |
| Viskosität der Basiskomponente / mPas | 3460 | 3020 | 3950 |

**Tabelle 6**

| Ausgeprüfte Mischungen und ermittelte Eigenschaften für Dubliermassen | | | | | | |
|---|---|---|---|---|---|---|
| | **Erfindungsbeispiele-Nr.** | | | | | |
| | **11** | **12** | **13** | **14** | **15** | **16** |
| Katalysatorkomponente (s. Tabelle 4) | DM-K1 | DM-K2 | DM-K4 | DM-K3 | DM-K5 | DM-K6 |
| Basiskomponente (s. Tabelle 5) | DM-B1 | DM-B1 | DM-B1 | DM-B2 | DM-B1 | DM-B3 |
| Mischungsverhältnis nach Gewicht | 1 : 1 | 1 : 1 | 1 : 1 | 1 : 1 | 1 : 1 | 1 : 1 |
| Aushärtungsbeginn / Sekunden | 195 | 675 | 135 | 315 | 240 | 215 |
| Aushärtungsende/Sekunden | 520 | 1800 | 420 | 855 | 780 | 760 |
| Shore A-Härte nach 24 Stunden | 23 | 19 | 18 | 22 | 16 | 25 |
| Reißfestigkeit/N | 13,5 | 15,4 | 9,5 | 16,7 | 10 | 14,1 |
| Reißdehnung/% | 131 | 192 | 127 | 201 | 160 | 170 |

**Tabelle 7**

| Zusammensetzung der Katalysatorkomponenten für Bißregistriermassen | | | |
|---|---|---|---|
| **Bestandteil** | **Bezeichnung der Katalysatorkomponenten** | | |
| | **Gew.-%** | | |
| | **BM-K1** | **BM-K2** | **BM-K3** |
| Umsetzungsprodukt von Borsäure und Salicylalkohol im Molverhältnis 1 : 2,1 | - | 10,40 | 12,15 |
| Umsetzungsprodukt von Borsäure und 5-Bromsalicylalkohol im Molverhältnis 1 : 1,95 | 19,80 | 4,70 | 2,75 |
| Salicylalkohol | - | - | 3,85 |
| Polypropylenoxid-diol mit einer Molmasse von 2100 g / Mol | 53,75 | 57,85 | 55,80 |
| Fällungskieselsäure (Sipernat D17) | 25,70 | 26,30 | 24,70 |
| Farbpaste, weiß | 0,75 | 0,75 | 0,75 |

**Tabelle 8**

| Zusammensetzung der Basiskomponenten für Bißregistriermassen | | |
|---|---|---|
| **Bestandteil** | **Bezeichnung der Basiskomponente** | |
| | **Gew.-%** | |
| | **BM-B1** | **BM-B2** |
| Gemisch aus Bisaziridinopolyethern mit einer mittleren Iminoäquivalentmasse von 3100, hergestellt aus einem Polyetherdiol, das aus Ethylenoxid-Einheiten und Tetrahydrofuran-Einheiten im Molverhältnis 1 : 3,6 besteht und einen Gehalt an cyclischen Ethern von 0,31 besitzt. | 58,10 | 25,00 |
| Gemisch aus Bisaziridinopolyethern mit einer mittleren Iminoäquivalentmasse von 1600, hergestellt aus einem Polyetherdiol, das aus Ethylenoxid-Einheiten und Tetrahydrofuran-Einheiten im Molverhältnis 1 : 3,4 besteht und einen Gehalt an cyclischen Ethern von 0,38 besitzt. | - | 29,75 |
| Diatomeenerde (Celatom MW 25) | 34,00 | 39,27 |
| Hydriertes Pflanzenöl | 5,00 | 4,20 |
| Dibenzyltoluol | 2,90 | 1,68 |

**Tabelle 9**

| Erfindungsgemäße Bißregistriermassen unter Verwendung der Katalysatorkomponenten gemäß Tabelle 7 und der Basiskomponenten gemäß Tabelle 8 und ermittelte Eigenschaften | | | | |
|---|---|---|---|---|
| | **Erfindungsbeispiele-Nr.** | | | |
| | **17** | **18** | **19** | **20** |
| Katalysatorkomponente (s. Tabelle 7) | BM-K1 | BM-K2 | BM-K2 | BM-K3 |
| Basiskomponente (s. Tabelle 8) | BM-B1 | BM-B1 | BM-B2 | BM-B2 |
| Mischungsverhältnis (nach Gewicht) K : B | 1 : 5,0 | 1 : 5,3 | 1 : 4,8 | 1 : 5,0 |
| Verarbeitungszeit / Sekunden | 50 | 45 | 70 | 80 |
| Mundentnahmezeit / Sekunden | 140 | 120 | 160 | 150 |
| Shore A-Härte (nach 24 h) | 71 | 73 | 81 | 83 |

## Patentansprüche

1. Elastomermasse auf der Grundlage von N-Alkylaziridinoverbindungen mit einer Basiskomponente, welche die Aziridinoverbindungen enthält, und mit einer Katalysatorkomponente, welche mindestens eine sauer wirkende Verbindung enthält, wobei beide Komponenten vor der Verwendung gemischt werden, **dadurch gekennzeichnet, daß** als eine sauer wirkende Verbindung der Katalysatorkomponente ein oder mehrere Borsäurekomplexe eingesetzt werden, die durch Umsetzung von Borsäure und/oder einem Borsäurederivat mit mindestens einer OH-funktiönellen Verbindung erhältlich sind, wobei die OH-Funktionen vollständig oder teilweise geschützt vorliegen können, und wobei diese Umsetzung entweder als vorgelagerte Reaktion zwischen Borsäure und/oder einem Borsäurederivat und mindestens einer solchen OH-funktionellen Verbindung oder während oder nach der Herstellung der Katalysatorkomponente oder durch Mischung der Katalysatorkomponerite mit der Basiskomponente, die dann mindestens eine solche OH-funktionelle Verbindung enthält, erfolgt, und wobei die mindestens eine OH-funktionelle Verbindung mindestens eine und bis zu 10 OH-Gruppen enthält und ausgewählt ist aus
(i) organischen Verbindungen, die mindestens eine 1,2-Dihydroxy- und/oder 1,3-Dihydroxygruppe enthalten,
(ii) Verbindungen, die mindestens eine phenolische OH-Gruppe besitzen,
(iii) α-Hydroxycarbonsäuren,
wobei die OH-funktionelle Verbindung die allgemeine Strukturformel 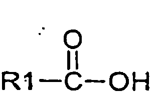 und bzw. oder 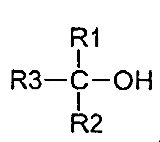 aufweist, wobei R1, R2 und R3 die gleichen oder verschiedene Reste repräsentieren, die Wasserstoff, aliphatische, cycloaliphatische, aromatische oder araliphatische Substituenten mit 1 bis 30 C-Atomen darstellen, in denen jeweils ein oder mehrere C-Atome durch -O-; -S-; 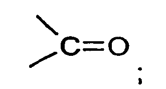 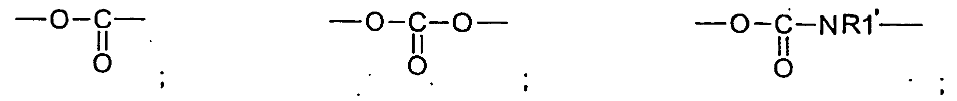 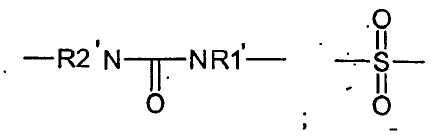 ersetzt sein können, wobei R1' und R2' gleich oder verschieden und aliphatische, cycloaliphatische, aromatische oder araliphatische zweiwertige Reste mit 1 bis 30 C-Atomen sind.

2. Elastomermasse gemäß Anspruch 1, **dadurch gekennzeichnet, daß** die Aushärtung der Aziridinoverbindungen durch Borsäurekomplexe erfolgt, die folgender allgemeinen Strukturformel 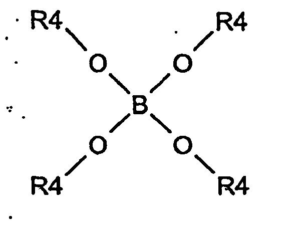 entsprechen,
wobei die Substituenten R4 unterschiedlich oder gleich sein können oder miteinander verbrückt sein können und R4 bedeuten kann: Wasserstoff, einen aliphatischen, cycloaliphatischen, aromatischen oder araliphatischen Rest mit 1 bis 30 C-Atomen und ein oder mehrere C-Atome durch -O-; -S-; 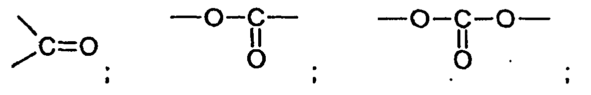 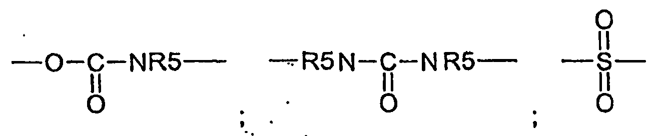 ersetzt sein können, wobei R5 Wasserstoff oder C1- bis C12-Alkyl bedeutet und R4 sowie R5 ein -oder mehrere Halogene, -CN, -OH, -SH, -COOH, -COO(C₁₋₁₈-Alkyl), -NO₂, SO₃H, Alkylthio-, Keto- sowie Aldehydgruppen als Substituenten tragen können.

3. Elastomermasse gemäß einem der Ansprüche 1 bis 2, **dadurch gekennzeichnet, daß** die Katalysatorkomponente 0,1 bis 100 Gew.-% von Borsäurekomplexen mit OH-funktionellen Verbindungen ggf. in einem Überschuß dieser OH-funktionellen Verbindungen enthält.

4. Elastomermasse gemäß einem der Ansprüche 1 bis 3, **dadurch gekennzeichnet, daß** das Verhältnis von Molanzahl Bor in der Katalysatorkomponente zur Zahl der Aziridinoäquivalente in der gemischten Zubereitung 1 : 1 bis 1 : 20 beträgt.

5. Elastomermasse nach einem der Ansprüche 1 bis 4, **dadurch gekennzeichnet, daß** die Borsäurekomplexe durch Umsetzung von Borsäure oder Borsäurederivaten mit Verbindungen hergestellt werden, die mindestens zwei OH-Gruppen enthalten, oder daß die Borsäurekomplexe durch Umsetzung von Borsäureestern mit Verbindungen hergestellt werden, die mindestens zwei OH-Gruppen enthalten.

6. Elastomermasse nach einem der Ansprüche 1 bis 5, **dadurch gekennzeichnet, daß** die Umsetzung der Borsäure oder des Borsäurederivats mit den OH-funktionellen Verbindungen vor der Formulierung der Katalysatorkomponente durchgeführt und der esterartige Borsäurekomplex als Bestandteil der Katalysatorkomponente eingesetzt wird, oder daß die Umsetzung der Borsäure oder des Borsäurederivats mit den OH-funktionellen Verbindungen während der Formulierung der Katalysatorkomponente erfolgt, oder daß die Umsetzung der Borsäure oder des Borsäurederivats mit den OH-funktionellen Verbindungen während und nach der Vermischung der Katalysatorkomponente mit der Basiskomponente erfolgt.

7. Elastomermasse nach einem der Ansprüche 1 bis 6, **dadurch gekennzeichnet, daß** die Bildung des härtungsauslösenden Borsäurekomptexes während und/oder nach der Vermischung der Katalysatorkomponente mit der Basiskomponente aus einem Borsäurederivat der Katalysatorkomponente und mindestens einer OH-funktbnellen Verbindung mit mindestens 2 OH-Gruppen der Basiskomponente erfolgt.

8. Elastomermasse nach einem der Ansprüche 1 bis 7, **dadurch gekennzeichnet, daß** die Bildung der härtungsauslösenden Borsäurekomplexe während und/oder nach der Vermischung der Katalysatorkomponente mit der Basiskomponente zumindestens teilweise aus einem Borsäurederivat, bevorzugt einem Borsäureester, der Katalysatorkomponente und mindestens einer OH-funktionellen Verbindung der Basiskomponente erfolgt.

9. Elastomermasse nach einem der Ansprüche 1 bis 8, **dadurch gekennzeichnet, daß** ein molares Verhältnis zwischen Borsäure und den OH-funktionellen Verbindungen von 1 : 0,1 bis 1 : 10, bevorzugt 1 : 1 bis 1 : 4 und besonders bevorzugt von 1 : 1,5 bis 1 : 3 eingestellt wird.

10. Elastomermasse gemäß Anspruch 1, **dadurch gekennzeichnet, daß** als Komplexbildner Brenzcatechin oder 2,3-Dihydroxynaphthalin eingesetzt wird, wobei der/die Phenylrest(e) ggf. weitere Substituenten wie Alkyl, Halogenid, Alkylester, Alkylether, Carboxyl und/oder Hydroxyl aufweisen kann/können, oder daß als Komplexbildner Salicylalkohol eingesetzt wird, wobei der Phenylrest ggf. weitere Substituenten wie Alkyl, Halogenid, Alkylester, Alkylether, Carboxyl und Hydroxyl aufweisen kann.

11. Elastomermasse gemäß einem der Ansprüche 1 bis 10, **dadurch gekennzeichnet, daß** als Komplexbildner Glycolsäure, Mandelsäure oder Benzilsäure eingesetzt werden.

12. Elastomermasse nach einem der Ansprüche 1 bis 11, **dadurch gekennzeichnet, daß** als Komplexbildner Verbindungen mit geschützten und bevorzugt silylierten OH-Gruppen eingesetzt werden.

13. Elastomermasse nach einem der. Ansprüche 1 bis 12, **dadurch gekennzeichnet, daß** mehrere Komplexbildner eingesetzt werden.

14. Elastomermasse nach einem der Ansprüche 1 bis 13, **dadurch gekennzeichnet, daß** Kombinationen von Borsäurekomplexen mit unterschiedlicher Struktur und Zusammensetzung eingesetzt werden.

15. Elastomermasse gemäß Anspruch 14, **dadurch gekennzeichnet, daß** als Komplexbildner 4-tert.-Butylbrenzcatechin in Kombination mit einer aliphatischen OH-funktionellen Verbindung eingesetzt wird.

16. Elastomermasse nach einem der Ansprüche 1 bis 15, **dadurch gekennzeichnet, daß** die Borsäurekomplexe zusammen mit anderen Startern und bevorzugt mit Sulfoniumstartern verwendet werden.

17. Verfahren zur Herstellung von Elastomermassen auf der Grundlage von N-Alkylaziridinoverbindungen mit einer Basiskomponente, welche die Aziridinoverbindungen enthält, und mit einer Katalysatorkomponente, welche mindestens eine sauer wirkende Verbindung enthält, wobei beide Komponenten vor der Verwendung gemischt werden, **dadurch gekennzeichnet, daß** als eine sauer wirkende Verbindung der Katalysatorkomponente ein oder mehrere Borsäurekomplexe eingesetzt werden, die durch Umsetzung von Borsäure und/oder einem Borsäurederivat mit mindestens einer OH-funktionellen Verbindung erhältlich sind, wobei die OH-Funktionen vollständig oder teilweise geschützt vorliegen können, und wobei diese Umsetzung entweder als vorgelagerte Reaktion zwischen Borsäure und/oder einem Borsäurederivat und mindestens einer solchen OH-funktionellen Verbindung oder während oder nach der Herstellung der Katalysatorkomponente oder durch Mischung der Katalysatorkomponente mit der Basiskomponente, die dann mindestens eine solche OH-funktionelle Verbindung enthält, erfolgt, und wobei die mindestens eine OH-funktionelle Verbindung mindestens eine und bis zu 10 OH-Gruppen enthält und ausgewählt ist aus
(i) organischen Verbindungen, die mindestens eine 1,2-Dihydroxy- und/oder 1,3-Dihydroxygruppe enthalten,
(ii) Verbindungen, die mindestens eine phenolische OH-Gruppe besitzen,
(iii) α-Hydroxycarbonsäuren,
wobei die OH-funktionelle Verbindung die allgemeine Strukturformel 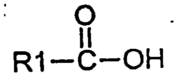 und bzw. oder 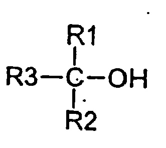 aufweist, wobei R1, R2 und R3 die gleichen oder verschiedene Reste repräsentieren, die Wasserstoff, aliphatische, cycloaliphatische, aromatische oder araliphatische Substituenten mit 1 bis 30 C-Atomen darstellen, in denen jeweils ein oder mehrere C-Atome durch -O-; -S-; 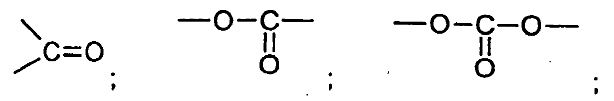 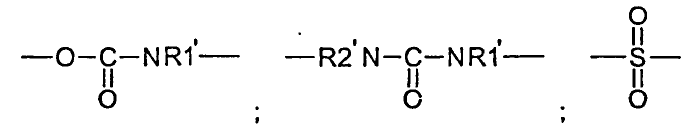 ersetzt sein können, wobei R1' und R2' gleich oder verschieden und aliphatische, cycloaliphatische, aromatische oder araliphatische zweiwertige Reste mit 1 bis 30 C-Atomen sind.

18. Verwendung von Elastomermassen nach einem der Ansprüche 1 bis 16 für die dentale Abformung, als Bißregistriermassen oder als Dubliermassen.

19. Kit, das die Basiskomponente und die Katalysatorkomponente gemäß einem der Ansprüche 1 bis 16 getrennt voneinander enthält.

## Claims

1. Elastomer material based on N-alkylaziridino compounds with a base component which contains the aziridino compounds and with a catalyst component which contains at least one acid-acting compound, both components being mixed before use, **characterized in that**, as an acid-acting compound of the catalyst component, one or more boric acid complexes are used which are obtainable by reaction of boric acid and/or a boric acid derivative with at least one OH-functional compound, the OH functions being able to be present wholly or partly protected, and this reaction being carried out either as an upstream reaction between boric acid and/or a boric acid derivative and at least one such OH-functional compound or during or after the preparation of the catalyst component or by mixing the catalyst component with the base component which then contains at least one such OH-functional compound, and the at least OH-functional compound containing at least one and up to 10 OH groups and being selected from
(i) organic compounds which contain at least one 1,2-dihydroxy and/or 1,3-dihydroxy group,
(ii) compounds which have at least one phenolic OH group, and
(iii)α-hydroxy-carboxylic acids,
wherein the OH-functional compound has the general structural formula 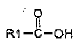 and/or 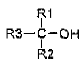 wherein R1, R2 and R3 represent the same or different radicals which are hydrogen, aliphatic, cycloaliphatic, aromatic or araliphatic substituents with 1 to 30 C atoms, in each of which one or more C atoms can be replaced by -O-; -S-; 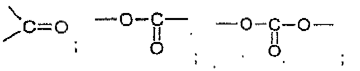 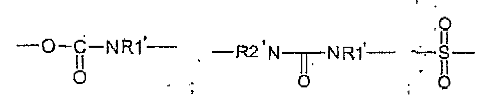 wherein R1' and R2' are the same or different and are aliphatic, cycloaliphatic, aromatic or araliphatic bivalent radicals with 1 to 30 C atoms.

2. Elastomer material according to Claim 1, **characterized in that** the curing of the aziridino compounds takes place through boric acid complexes which correspond to the following general structural formula 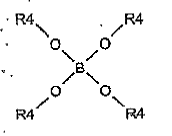 wherein the substituents R4 can be different or the same or bridged with each other and R4 can mean: hydrogen, an aliphatic, cycloaliphatic, aromatic or araliphatic radical with 1 to 30 C atoms and one or more C atoms can be replaced by -O-; -S-; 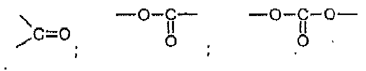 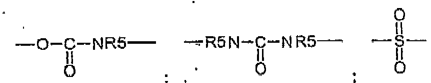 wherein R5 is hydrogen or C1 to C12 alkyl and R4 and also R5 can carry one or more halogens, -CN, -OH, -SH, -COOH, -COO(C₁₋₁₈ alkyl), -NO₂, SO₃H, alkylthio, keto and also aldehyde groups as substituents.

3. Elastomer material according to one of Claims 1 to 2, **characterized in that** the catalyst component contains 0.1 to 100 wt.-% of boric acid complexes with OH-functional compounds optionally in an excess of these OH-functional compounds.

4. Elastomer material according to one of Claims 1 to 3, **characterized in that** the ratio of number of moles of boron in the catalyst component to the number of aziridino equivalents in the mixed preparation is 1:1 to 1:20.

5. Elastomer material according to one of Claims 1 to 4, **characterized in that** the boric acid complexes are prepared by reaction of boric acid or boric acid derivatives with compounds which contain at least two OH groups or **in that** the boric acid complexes are prepared by reaction of boric acid esters with compounds which contain at least two OH groups.

6. Elastomer material according to one of Claims 1 to 5, **characterized in that** the reaction of the boric acid or the boric acid derivative with the OH-functional compounds is carried out before the formulation of the catalyst component and the ester-like boric acid complex is used as a constituent of the catalyst component, or **in that** the reaction of the boric acid or the boric acid derivative with the OH-functional compounds takes place during the formulation of the catalyst component, or **in that** the reaction of the boric acid or the boric acid derivative with the OH-functional compounds takes place during and after the mixing of the catalyst component with the base component.

7. Elastomer material according to one of Claims 1 to 6, **characterized in that** the formation of the curing-triggering boric acid complex takes place during and/or after the mixing of the catalyst component with the base component from a boric acid derivative of the catalyst component and at least one OH-functional compound with at least 2 OH groups of the base component.

8. Elastomer material according to one of Claims 1 to 7, **characterized in that** the formation of the curing-triggering boric acid complexes takes place during and/or after the mixing of the catalyst component with the base component at least partly from a boric acid derivative, preferably a boric acid ester, of the catalyst component and at least one OH-functional compound of the base component.

9. Elastomer material according to one of Claims 1 to 8, **characterized in that** a molar ratio between boric acid and the OH-functional compounds of from 1:0.1 to 1:10, preferably 1:1 to 1:4 and with particular preference from 1:1.5 to 1:3 is set.

10. Elastomer material according to Claim 1, **characterized in that**, as complexing agent, pyrocatechol or 2,3-dihydroxynaphthalene is used, the phenyl radical(s) optionally being able to contain further substituents such as alkyl, halide, alkyl ester, alkyl ether, carboxyl and/or hydroxyl, or **in that** salicylic alcohol is used as complexing agent, the phenyl radical optionally being able to contain further substituents such as alkyl, halide, alkyl ester, alkyl ether, carboxyl and hydroxyl.

11. Elastomer material according to one of Claims 1 to 10, **characterized in that**, as complexing agents, glycolic acid, mandelic acid or benzilic acid are used.

12. Elastomer material according to one of Claims 1 to 11, **characterized in that**, as complexing agents, compounds with protected and preferably silylated OH groups are used.

13. Elastomer material according to one of Claims 1 to 12, **characterized in that** several complexing agents are used.

14. Elastomer material according to one of Claims 1 to 13, **characterized in that** combinations of boric acid complexes with varying structure and composition are used.

15. Elastomer material according to Claim 14, **characterized in that**, as complexing agent, 4-tert-butylpyrocatechol is used in combination with an aliphatic OH-functional compound.

16. Elastomer material according to one of Claims 1 to 15, **characterized in that** the boric acid complexes are used together with other starters and preferably with sulphonium starters.

17. Process for the preparation of elastomer materials based on N-alkylaziridino compounds with a base component which contains the aziridino compounds and with a catalyst component which contains at least one acid-acting compound, both components being mixed before use, **characterized in that**, as an acid-acting compound of the catalyst component, one or more boric acid complexes are used which are obtainable by reaction of boric acid and/or a boric acid derivative with at least one OH-functional compound, the OH functions being able to be present wholly or partly protected, and this reaction being carried out either as an upstream reaction between boric acid and/or a boric acid derivative and at least one such OH-functional compound or during or after the preparation of the catalyst component or by mixing the catalyst component with the base component which then contains at least one such OH-functional compound, and the at least one OH-functional compound containing at least one and up to 10 OH groups and being selected from
(i) organic compounds which contain at least one 1,2-dihydroxy and/or 1,3-dihydroxy group,
(ii) compounds which have at least one phenolic OH group, and
(iii)α-hydroxy-carboxylic acids,
wherein the OH-functional compound has the general structural formula 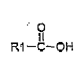 and/or 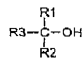
wherein R1, R2 and R3 represent the same or different radicals which are hydrogen, aliphatic, cycloaliphatic, aromatic or araliphatic substituents with 1 to 30 C atoms, in each of which one or more C atoms can be replaced by -O- ; -S- ; 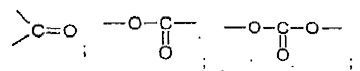 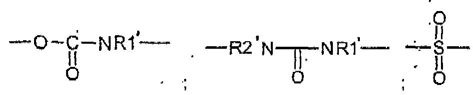 wherein R1' and R2' are the same or different and are aliphatic, cycloaliphatic, aromatic or araliphatic bivalent radicals with 1 to 30 C atoms.

18. Use of elastomer materials according to one of Claims 1 to 16 for dental impression, as bite-registration materials or as doubling materials.

19. Kit which contains the base component and the catalyst component according to one of Claims 1 to 16 separately from each other.

## Revendications

1. Matières élastomères à base de composés de N-alkylaziridine comprenant un composant de base, qui contient les composés d'aziridine, et un composant catalyseur contenant au moins un composé à action acide, les deux composants étant mélangés avant l'utilisation, **caractérisé en ce que** l'on met en oeuvre en tant que composé à action acide un ou plusieurs complexes de bore, que l'on peut obtenir par la réaction d'acide borique et/ou d'un dérivé d'acide borique avec au moins un composé à fonctionnalité OH, les fonctions OH pouvant être totalement ou partiellement protégées, et cette réaction se faisant, soit en tant que réaction préalable entre l'acide borique et/ou un dérivé d'acide borique et au moins un tel composé à fonctionnalité OH, soit pendant ou après la préparation du composant catalyseur, soit par mélange du composant catalyseur avec le composant de base, qui contient alors au moins un tel composé à fonctionnalité OH, et le au moins un composé à fonctionnalité OH contenant au moins un et jusqu'à 10 groupes OH et est choisi parmi
(i) des composés organiques contenant au moins un groupe 1,2-dihydroxy et/ou 1,3-dihydroxy,
(ii) des composés possédant au moins un groupe OH phénolique,
(iii) des acides α-hydroxycarboxyliques,
où le composé à fonctionnalité OH présente la structure générale 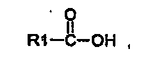 et/ou 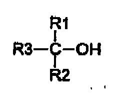 où R1, R2 et R3 représentent des radicaux identiques ou différents, qui sont de l'hydrogène, des substituants aliphatiques, cycloaliphatiques, aromatiques ou araliphatiques comportant de 1 à 30 atomes de C, dans lesquels à chaque fois un ou plusieurs atomes de C peuvent être remplacés par -O- -S- 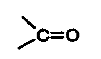 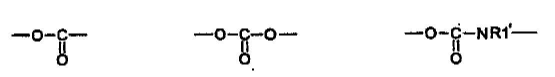 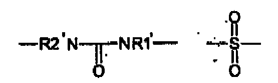 où R1' et R2' représentent des radicaux divalents aliphatiques, cycloaliphatiques, aromatiques ou araliphatiques identiques ou différents et comportant de 1 à 30 atomes de C.

2. Matières élastomères selon la revendication 1, **caractérisées en ce que** le durcissement des composés d'aziridine se fait via des complexes de bore, qui correspondent à la formule structurelle générale suivante 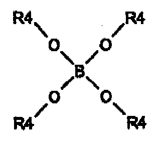 dans laquelle les substituants R4 peuvent être identiques ou différents ou être pontés entre eux et R4 peut représenter de l'hydrogène, un radical aliphatique, cycloaliphatique, aromatique ou araliphatique comportant de 1 à 30 atomes de C et où un ou plusieurs atomes de C peuvent être remplacés par 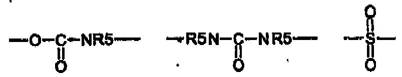 -O-, -S- 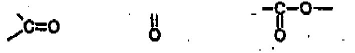 où R5 représente de l'hydrogène ou un radical alkyle en C₁ à C₁₂ et R4 et R5 peuvent porter en tant que substituant(s) un ou plusieurs halogène(s), -CN, -OH, -SH, -COOH, -COO(alkyle en C₁ à C₁₈), -NO₂, SO₃H, ou des groupes alkylthio, cétone et aldéhyde.

3. Matières élastomères selon l'une quelconque des revendications 1 à 2, **caractérisées en ce que** le composant catalyseur contient de 0,1 à 100% en poids de complexes de bore avec des composés à fonctionnalité OH, le cas échéant en un excès de ces composés à fonctionnalité OH.

4. Matières élastomères selon l'une quelconque des revendications 1 à 3, **caractérisées en ce que** la proportion du nombre de moles de bore dans le composant catalyseur au nombre des équivalents aziridine dans la préparation mélangée est de 1:1 à 1:20.

5. Matières élastomères selon l'une quelconque des revendications 1 à 4, **caractérisées en ce que** les complexes de bore sont préparés par réaction d'acide borique ou de dérivés d'acide borique avec des composés contenant au moins deux groupes OH, ou **en ce que** les complexes de bore sont préparés par réaction d'esters d'acide borique avec des composés contenant au moins deux groupes OH.

6. Matières élastomères selon l'une quelconque des revendications 1 à 5, **caractérisées en ce que** la réaction de l'acide borique ou du dérivé d'acide borique avec les composés à fonctionnalité OH est entreprise avant la formulation du composant catalyseur et que le complexe de bore de type ester est mis en oeuvre en tant que constituant du composant catalyseur, ou que la réaction de l'acide borique ou du dérivé d'acide borique avec les composés à fonctionnalité OH a lieu pendant la formulation du composant catalyseur, ou que la réaction de l'acide borique ou du dérivé d'acide borique avec les composés à fonctionnalité OH a lieu pendant ou après le mélange du composant catalyseur au composant de base.

7. Matières élastomères selon l'une quelconque des revendications 1 à 6, **caractérisées en ce que** la formation du complexe d'acide borique déclenchant le durcissement a lieu pendant et/ou après le mélange du composant catalyseur au composant de base à partir d'un dérivé d'acide borique du composant catalyseur et d'au moins un composé à fonctionnalité OH comportant au moins 2 groupes OH du composant de base.

8. Matières élastomères selon l'une quelconque des revendications 1 à 7, **caractérisées en ce que** la formation du complexe d'acide borique déclenchant le durcissement a lieu, pendant et/ou après le mélange du composant catalyseur au composant de base, à partir d'au moins en partie d'un dérivé d'acide borique, de préférence un ester de l'acide borique, du composant catalyseur et d'au moins un composé à fonctionnalité OH du composant de base.

9. Matières élastomères selon l'une quelconque des revendications 1 à 8, **caractérisées en ce que** l'on ajuste une proportion molaire entre l'acide borique et les composés à fonctionnalité OH de 1:0,1 à 1:10, de préférence de 1:1 à 1:4 et plus préférentiellement de 1:1,5 à 1:3.

10. Matières élastomères selon la revendication 1, **caractérisées en ce que** l'on met en oeuvre en tant que complexant de la pyrocatéchine ou du 2,3-dihydroxynaphtalène, où le(s) reste(s) phényle peu(ven)t présenter le cas échéant d'autres substituants tels que des radicaux alkyle, halogénure, ester alkylique, éther alkylique, carboxyle et/ou hydroxyle, ou que l'on met en oeuvre en tant que complexant de l'alcool salicylique, où le reste phényle peut présenter le cas échéant d'autres substituants tels que des radicaux alkyle, halogénure, ester alkylique, éther alkylique, carboxyle et hydroxyle.

11. Matières élastomères selon l'une quelconque des revendications 1 à 10, **caractérisées en ce que** l'on met en oeuvre en tant que complexant de l'acide glycolique, de l'acide mandélique ou de l'acide benzilique.

12. Matières élastomères selon l'une quelconque des revendications 1 à 11, **caractérisées en ce que** l'on met en oeuvre en tant que complexants des composés comportant des groupes OH protégés et de préférence silylés.

13. Matières élastomères selon l'une quelconque des revendications 1 à 12, **caractérisées en ce que** l'on met en oeuvre plusieurs complexants.

14. Matières élastomères selon l'une quelconque des revendications 1 à 13, **caractérisées en ce que** l'on met en oeuvre des combinaisons de complexes d'acide borique de structure et de composition différentes.

15. Matières élastomères selon la revendication 14, **caractérisées en ce que** l'on met en oeuvre en tant que complexants de la 4-tert.-butylpyrocatéchine en combinaison avec un composé aliphatique à fonctionnalité OH.

16. Matières élastomères selon l'une quelconque des revendications 1 à 15, **caractérisées en ce que** les complexes d'acide borique sont utilisés en même temps que d'autres amorces et de préférence avec des amorces de sulfonium.

17. Procédé de préparation de matières élastomères à base de composés de N-alkylaziridine avec un composant de base, contenant les composés d'aziridine, et avec un composant catalyseur, qui contient au moins un composé à action acide, les deux composants étant mélangés avant l'utilisation, **caractérisé en ce que** l'on met en oeuvre en tant que composé à action acide du composant catalyseur un ou plusieurs complexes d'acide borique, que l'on peut obtenir par réaction d'acide borique et/ou d'un dérivé d'acide borique avec au moins un composé à fonctionnalité OH, où les fonctions OH peuvent être protégées totalement ou partiellement, et où cette réaction a lieu, soit en tant que réaction préalable entre l'acide borique et/ou un dérivé d'acide borique et au moins un tel composé à fonctionnalité OH, soit pendant ou après la préparation du composant catalyseur ou par mélange du composant catalyseur avec le composant de base, qui contient alors un tel composé à fonctionnalité OH, et où le au moins un composé à fonctionnalité OH contient d'au moins un à 10 groupes OH et est choisi parmi
(i) des composés organiques contenant au moins un groupe 1,2-dihydroxy et/ou 1,3-dihydroxy,
(ii) des composés possédant au moins un groupe OH phénolique,
(iii) des acides α-hydroxycarboxylique,
où le composé à fonctionnalité OH présente la structure générale 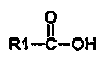 et/ou 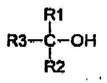 où R1, R2 et R3 représentent des radicaux identiques ou différents, qui sont de l'hydrogène, des substituants aliphatiques, cycloaliphatiques, aromatiques ou araliphatiques comportant de 1 à 30 atomes de C, dans lesquels à chaque fois un ou plusieurs atomes de C peuvent être remplacés par -O- -S- 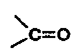 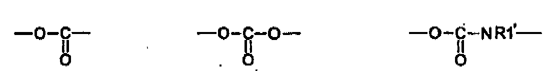 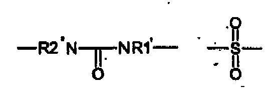 où R1' et R2' représentent des radicaux divalents aliphatiques, cycloaliphatiques, aromatiques ou araliphatiques identiques ou différents et comportant de 1 à 30 atomes de C.

18. Utilisation de matières élastomères selon l'une quelconque des revendications 1 à 16 pour le moulage dentaire, en tant que masses pour empreintes ou en tant que masses de duplication.

19. Trousse comprenant le composant de base et le composant catalyseur selon l'une quelconque des revendications 1 à 16 séparés l'un de l'autre.
